# EUROPEAN PATENT APPLICATION

(11) **EP 2 044 933 A1**
(43) Date of publication of application: **08.04.2009**
(21) Application number: 07019527.6
(22) Date of filing: 05.10.2007
(51) Int. Cl.: A61K 9/22, A61K 9/48, A61K 31/55

(54) **Multi particulate matrix system containing galantamine**

(71) Applicant: KRKA, D.D., Novo Mesto, 8501 Novo mesto (SI)
(72) Inventor: Preskar, Maja, 8270 Krsko (SI); German, Tamara, 8000 Novo mesto (SI); Vrecer, Franc, 8351 Straza pri Novem mestu (SI); Dobrovoljc, Katarina, 1241 Kamnik (SI); Kroselj, Vesna, 8310 Sentjernej (SI)
(74) Representative: HOFFMANN EITLE

(57) **Abstract**

The present invention provides for a multi particulate matrix system (MPMS) comprising at least one type of particles which contains galantamine or a pharmacologically acceptable salt thereof as an active substance, wherein said at least one type of particles provides for prolonged release of said active substance, and wherein all particles present in the MPMS are uncoated. The present invention further provides for a process for preparing a MPMS.

## Description

### Field of the Invention

The present invention provides for a multi particulate matrix system (MPMS) comprising at least one type of particles which contains galantamine or a pharmacologically acceptable salt thereof as an active substance, wherein said at least one type of particles provides for a prolonged release of said active substance, and wherein all particles present in the MPMS are uncoated. The invention further relates to a process of making such a MPMS and a pharmaceutical composition containing such a MPMS. The invention particularly relates to suitable oral formulations comprising the active ingredient galantamine hydrobromide, using such a technology.

### Background of the Invention

Prolonged release formulations offer the possibility of reducing dosage regimes for drugs, especially for those drugs administered orally to patients, by prolonging the time period during which pharmacologically effective levels of the active ingredient are present in the body. Prolonged release formulations thereby result in a better assurance of compliance, reduction of severity and frequency of side effects, since the drug level in the blood is more constant, and of drug level fluctuations associated with conventional immediate release formulations administered several times a day are avoided.

Alzheimer disease is affecting 8% to 10% of people older than 65 and as many as 40% of those older than 85 years. It has enormous associated costs of as much as 80 to 100 billion USD per year, leads to psychiatric symptoms of burnout in caregivers and is the common cause of institutionalization of elderly persons. The prevalence of dementia in the nursing home population has been estimated at 25% to 74%.

Suspicion of dementia is warranted whenever an elderly patient presents a memory complaint, difficulties with activities of daily living, personality change or a new behavioural problem. The life expectancy of the typical Alzheimer disease patient is 4 to 6 years.

Estimations made with representative of a therapeutic group of acetyl cholinesterase inhibitors, such as galantamine, showed lower costs with active therapy and lower risk inpatient hospitalization. Due to nature of disease (e.g. progressive worsening of memory, deficits in two or more cognitive areas etc.) drugs of one daily dosage are more favourable, especially in stages of mild to moderate Alzheimer disease in order to improve compliance and therefore prolong the time for requiring care or even institutionalization of the patients. By improving the compliance therefore not only symptoms and signs are improved, however, also the quality of life of the patient is sustained or improved. One daily dosage is also convenient to caregivers of the patients at home, hospitals or nursing homes.

To assure a substantially constant drug level in the blood and to ensure at the same time that the drug displays its effect as early as possible after administration of the drug, it is desirable to provide a system showing a combined release behaviour. That is, the system should both release therapeutically relevant concentrations of the active ingredient immediately after drug uptake and ensure that such concentrations are maintained over a prolonged period of time, when compared with a pure immediate-release formulation. In principle, such systems are state of the art: Ying-huan Li et al. (Modulation of combined-release behaviours from a novel "tablets-in-capsule system", Journal of Controlled Release 2004, 95(3), 381-389) describes a multifunctional and multiple unit system, which contains versatile mini-tablets in a hard gelatine capsule, developed by preparing Rapid-release Mini-Tablets (RMTs), coated Sustained-release Mini-Tablets (SMTs), Pulsatile Mini-Tablets (PMTs), and Delayed-onset Sustained-release Mini-Tablets (DSMTs), each with various lag times of release. Based on the combinations of mini-tablets, multiple pulsatile drug delivery systems (DDS), site-specific DDS, slow/quick DDS, quick/slow DDS, and zero-order DDS a velocity-time curve could be obtained instead of the cumulative percentage drug release profile.

In US 6,110,494 a sustained release cisapride oral dosage formulation suitable for one-daily administration is disclosed, comprising a plurality of mini-tablets containing cisapride or a salt thereof with an organic acid. Said sustained release oral formulation is reported to be capable of releasing cisapride at different sites along the gastrointestinal tract. The mini-tablets include a proportion of immediate release tablets and a proportion of tablets which release cisapride in response to the pH environment at a given site in the distal regions of the gastrointestinal tract. Cisapride or a salt thereof is embedded in a matrix of hydrophilic polymer, which is then coated with a pH dependent polymer to provide the desired sustained release.

However, both the systems of Li et al. and the system of US 6,110,494 are relatively complicated in terms of their design and thus expensive.

Controlled release formulations comprising only one species of solid compacts are also known and include the following: Carla M. Lopes, et al. (Directly Compressed Mini Matrix Tablets Containing Ibuprofen: Preparation and Evaluation of Sustained Release, Drug Development and Industrial Pharmacy 2006, 32(1), pp. 95-106) describe directly compressed mini tablets containing ibuprofen and either hydroxypropylmethylcellulose (HPMC) or ethylcellulose (EC) as release controlling agent. By changing the polymer concentration, the ibuprofen release was modified. In identical quantities, EC produced a greater sustaining release effect than HPMC. Different grades of viscosity of HPMC did not modify ibuprofen release. For EC formulations, the contribution of diffusion was predominant in the ibuprofen release process. For HPMC preparations, the drug release approached zero-order during a period of 8 h.

In WO 2005/084639 an oral delivery system for Class II drugs is described. The formulation is a controlled release or an immediate release formulation. The immediate release formulation contains a Class II drug together with a hydrophobic polymer. The drug and the polymer are co-dissolved in a common solvent, from which solid particles are formed containing the drug dispersed in the form of small particles in a polymeric matrix. The particles are stable against aggregation, and can be put into capsules or tableted for administration.

The controlled release formulations of WO 2005/084639 are in the form of tablets, capsules, mini-tabs, microparticulates or osmotic pumps. Enhancement of oral uptake of the drug by the use of bioadhesive polymers occurs through increased dissolution kinetics due to stable micronization of the drug, rapid release of the drug from the polymer in the GI tract and prolonged GI transit due to bioadhesive properties of the polymers.

US 6,514,531 is directed to controlled-release dosage forms of zolpidem or salts thereof adapted to release zolpidem over a predetermined time period, according to a biphasic profile of dissolution, where the first phase is an immediate release phase and the second phase is a prolonged release phase.

State of the art that specifically pertains to the drug galantamine, but not necessarily limited to controlled release formulations, includes the following documents:
EP 0 915 701 B1 is concerned with a fast-dissolving tablet for oral administration comprising as an active ingredient a therapeutically effective amount of galantamine hydrobromide and a pharmaceutically acceptable carrier. The carrier comprises a spray dried mixture of lactose monohydrate and microcrystalline cellulose as a diluent, a crospovidone or croscarmellose as an insoluble or poorly soluble cross-linked polymer disintegrant and glidants which do not comprise talc; moreover this patent discloses a direct compression process of preparing fast-dissolving tablets.
EP 1 140 105 B1 relates to controlled release compositions for oral administration comprising galantamine. Said controlled release composition comprises particles comprising galantamine or a pharmaceutically acceptable acid addition salt thereof, a water soluble pharmaceutically acceptable excipient and optionally other pharmaceutically acceptable excipients. The particles are coated by a release rate controlling membrane coating.
WO 2005/065662 A1 is directed to an immediate release pharmaceutical solid dosage formulation comprising galantamine or a pharmaceutically acceptable salt thereof and a pharmaceutically acceptable excipient, with the proviso that the formulation does not contain microcrystalline cellulose.
WO 2005/065661 A2 relates to an immediate and extended release dosage formulation comprising galantamine or a pharmaceutically acceptable salt thereof and a pharmaceutically acceptable carrier, wherein the carrier is substantially free of a spray dried mixture of lactose monohydrate and microcrystalline cellulose; and wherein the formulation exhibits a dissolution profile such that after 0.5 h at least about 80% of the galantamine or galantamine salt is released after combining the dosage formulation with 500 ml purified water at 37°C in Apparatus 2.

An object underlying the present invention is to provide a further simplified and thus inexpensive solid pharmaceutical formulation that provides for combined release, i.e. both fast onset and continuous release of the active ingredient galantamine, particularly galantamine hydrobromide. In contrast to the pharmaceutical formulations according to the prior art described above, the present invention relates to a multi particulate matrix system comprising at least one type of particles, of which at least one enables a prolonged release of an active substance, but which consists only of uncoated particles. Such an uncoated solid dosage form offers the advantage of an easier scale-up, better cost-effectiveness due to lower cost of production, no need for sophisticated equipment and less time-consuming production processes. The uncoated particles according to the present invention can be prepared by direct compression which represents a very simple and easy method.

### Summary of the Invention

Thus, in one aspect, the present invention provides a multi particulate matrix system (MPMS) comprising at least one type of particles which contains galantamine or a pharmacologically acceptable salt thereof as an active substance, wherein said at least one type of particles provides for a prolonged release of said active substance, and wherein all particles present in the MPMS are uncoated.

The MPMS according to the present invention may further contain a second type of particles. The second type of particles either provides for immediate or prolonged release of the active substance.

The active substance is preferably galantamine hydrobromide.

In a second aspect, the present invention provides for a pharmaceutical solid dosage formulation comprising the multi particulate matrix system described above.

In a third aspect, the present invention provides for a process for the manufacture of a multi particulate matrix system as described above wherein each particle type of the multi particulate matrix system is prepared by a process comprising a step selected from the group consisting of dry granulation, wet granulation, melt granulation and direct compression, followed by mixing of the respective particle types.

In a further aspect, the present invention provides for a process for forming a pharmaceutical solid dosage formulation as described above comprising the step of melt granulation of a mixture containing at least the active ingredient and a binder to obtain granules, followed by either directly incorporating these granules into capsules or sachets, or by compressing them into tablets.

### Brief Description of Drawings

- **Fig. 1:**: Morphology of galantamine HBr used in Ex. 1
- **Fig. 2:**: Morphology of galantamine HBr used in Ex. 2
- **Fig. 3:**: Morphology of galantamine HBr used in Ex. 3
- **Fig. 4:**: Morphology of galantamine HBr used in Ex. 4
- **Fig. 5:**: Morphology of galantamine HBr used in Ex. 5

### Detailed Description

In one aspect, the present invention provides for a multi particulate matrix system (MPMS) comprising at least one type of particles which contains galantamine or a pharmacologically acceptable salt thereof as an active substance, wherein said at least one type of particles provides for prolonged release of said active substance, and wherein all particles present in the MPMS are uncoated.

"Active ingredient" in the context of this invention means galantamine or a pharmaceutically acceptable salt thereof.

"One type of particles" in the context of this invention means that the particles have the same composition and approximately the same size and shape, e.g. 2, 3 or more mini tablets produced by the same process.

"Prolonged release" in the context of this invention means that the dosage formulation exhibits a dissolution profile such that after 2 hours 20 to 60% of the active ingredient originally contained in a particle type is released, after 4 hours 40 to 80% of the active ingredient contained in a particle type is released, and after 12 hours more than 70% of the active ingredient contained in a particle type is released after dissolving the dosage formulation in 500 ml USP buffer pH 6.8 at 37°C in an Apparatus 1 (Ph.Eur. and USP, baskets, 100 rpm). Unless stated otherwise, all percentages given herein are by weight. Due to nature of the preparation process of the mini or micro tablets the dissolution values underlie certain variations (cf. Ph.Eur.). Values above or below 5% of the indicated constraints given above are to be understood as falling within the indicated range(s).

In a preferred embodiment 5% or more of galantamine or a pharmaceutically acceptable salt thereof, are still present after 12h and even more preferred after 16h. In a preferred embodiment 80% or more of galantamine or a pharmaceutically acceptable salt thereof are released after 12h and even more preferred after 16h.

"Immediate release" in the context of this invention means that 50% or more of the active ingredient contained in a particle type are dissolved under the above conditions after 1h.

The MPMS of the present invention may contain two, three, four or more types of particles that differ in their compositions so as to provide for a different release of the active ingredient. The MPMS of the present invention may differ either in the amount of active ingredient contained in the respective particle types or the types and/or amounts of excipients in the respective particle types. In a further preferred embodiment of the present invention, the two, three or more particle types respectively differ in both the amount of active ingredient and the amount of excipients contained therein.

At least one type of particles in the MPMS according to the present invention provides for a prolonged release of the active ingredient.

The MPMS according to the present invention may further contain a second type of particles. The second type of particles either provides for immediate or prolonged release of the active substance.

However, the release of active ingredient by each particle type must in any case be different, i.e. one type of particles should release the active ingredient faster than the other(s).

The active substance contained in the particles of the MPMS may be present in the form of a salt, hydrate or solvate thereof. It may further be present in either anhydrous or solvated form, in a crystalline or non-crystalline form such as a polymorphic, pseudopolymorphic or amorphous form. The terms "active ingredient" and "active substance", which are used synonymously in this application, should be understood to include any such salt, solvate or crystal form of the respective active ingredient.

In a preferred embodiment the active substance is galantamine hydrobromide.

The amount of active substance present in each particle type of the MPMS is 5-90% w/w, 8-60% w/w, and more preferably 10-30% w/w.

The amount of active substance present in each particle type of the MPMS is 1.5-50 mg, preferably 1-30 mg, and more preferably 1.5-15 mg.

In a preferred embodiment of the invention, at least one type of the particles contained in the MPMS is non-spherical. The particles of the MPMS of the present invention may for example be present in the form of micro or mini tablets having a diameter of 1 mm to 5 mm.

In a further embodiment of the invention, the particles of the MPMS may have the following characteristics:
- Weight:: 5-100 mg, preferably 10-80 mg, more preferably 10-60 mg
- Hardness:: 5-80 N, preferably 10-60 N, more preferably 15-50 N
- Diameter:: 1-6 mm, preferably 1-5 mm, more preferably 1.5-5 mm
- Shape:: round, oval, with or without bevelled edges, with or without imprint
- Thickness:: 1-6 mm, preferably 1.2-5 mm, more preferably 1.4-3 mm
- Face surface:: flat or convex
- Colour:: any colour is suitable, white is preferably

The particles of the MPMS of the present invention may, in addition to the active ingredient, further comprise one or more pharmaceutically acceptable excipient(s). Suitable excipients are selected from the group consisting of a diluent, a binder, a disintegrant, a gelling agent, a non-swellable polymer, a lipid substance, a surfactant, a lubricant and a glidant.

In a preferred embodiment the diluent is selected from the group consisting of microcrystalline cellulose, powdered cellulose, composite materials combining crystalline cellulose with lactose (Cellactose, Tablettose), guar gum (Avicel CE15) or silicified cellulose (Prosolv), calcium hydrogen phosphate in anhydrous and hydrated form, various types of sugars such as lactose (anhydrous and monohydrate), compressible sugar, fructose, dextrates, sugar alcohols such as mannitol, sorbitol, maltitol, xylitol, lactitol, or other sugars such as saccharose, raffinose, trehalose, fructose or mixture thereof, calcium carbonate, calcium lactate or mixture thereof. In a more preferred embodiment, the diluent is lactose (Tablettose).

In a preferred embodiment the binder is selected from the group consisting of polyvinylpyrrolidone, Kollidon SR, microcrystalline cellulose, cellulose ether, hydroxyethylcellulose, hydroxypropylcellulose, hydroxypropylmethylcellulose, Methocel® K4M, starch, pregelatinised starch, or polymethacrylate, or mixtures thereof. In a more preferred embodiment, the binder is selected from Kollidon SR and Methocel® K4M.

In a preferred embodiment the disintegrant is selected from the group consisting of crospovidone, starch, pregelatinised starch, sodium starch glycollate, microcrystalline cellulose, carboxymethylcellulose sodium (CMC-Na) or calcium (CMC-Ca), cross-linked CMC-Na, polacrilin potassium, low-substituted hydroxypropylcellulose or mixtures thereof.

In a preferred embodiment the gelling agent is selected from the group consisting of hydroxypropylmethylcellulose (HPMC), hydroxypropylcellulose, hydroxycellulose phthalate, poly(ethyleneoxide), polylactic acid, xanthan gum, alginates, sodium and calcium carboxymethylcellulose, carragheenan, carbomer, carbopol, methylhydroxyethylcellulose, propylhydroxyethylcellulose, polyHEMA, methylcellulose, alginates and other swellable polymers.

In a preferred embodiment the non-swellable polymer is selected from the group consisting of water insoluble, non-swelling polymers such as: ethyl cellulose, cellulose acetate propionate, cellulose acetate, poly(ethyl acrylate, methyl methacrylate, trimethylammonioethyl methacrylate chloride)1:2:0.1, sold as Eudragit^{®} RS 100 poly(ethyl acrylate, methyl methacrylate, trimethylammonioethyl methacrylate chloride) 1:2:0.2 copolymer, commercially available as Eudragit^{®} RL, polyvinylpyrrolidone acetate, Eudragit^{®} RS PO, polyvinyl chloride, polyvinyl acetate, and polyethylene. In a more preferred embodiment, the non-swellable polymer is Eudragit^{®} RS PO.

In a preferred embodiment the lipid substance is selected from the group consisting of fatty alcohols with 10-18 C atoms such as stearol, palmitol, esters and ether of fatty acids such as mono, di and triglycerides with 10-18 C atoms in the fatty acid residue.

The surfactant, if present, is selected from the group consisting of anionic surfactants, ampholytic surfactants, nonionic surfactants and cationic surfactants.

In a preferred embodiment the anionic surfactant is selected from the group consisting of organic sulphonates (RSO₃⁻) or sulphates (ROSO₃⁻), potassium laurate, CH₃(CH₂)₁₀COO⁻K⁺, and sodium lauryl sulphate, CH₃(CH₂)₁₁SO₄⁻Na⁺. The most preferred anionic surfactant is sodium lauryl sulphate.

In a preferred embodiment the cationic surfactant is selected from the group consisting of organic quaternary ammonium halides, R₄N⁺Cl⁻, cetrimide, a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula [C₆H₅CH₂N⁺(CH₃)₂R]Cl⁻, where R represents a mixture of alkyls from C₈H₁₇ to C₁₈H₃₇.

In a preferred embodiment the ampholytic surfactant is selected from sulfobetaines, RN⁺(CH₃)₂CH₂CH₂SO₃⁻), N-Dodecyl-N,N-Dimethylbetaine, C₁₂H₂₅N⁺(CH₃)₂CH₂COO⁻.

In a preferred embodiment the nonionic surfactant contains hydroxyl or polyoxyethylene (OCH₂CH₂O⁻) groups, and more preferably it is selected from polyoxyethylated glycol monoethers, cetomacrogol, sorbitan esters (Spans) and polysorbates (Tweens), or from polyoxyethylene-polyoxypropylene copolymers.

In a preferred embodiment the lubricant and the glidants are selected from the group consisting of stearic acid, magnesium stearate, magnesium palmitate, magnesium oleate, hydrogenated vegetable oil, hydrogenated castor oil, talc, sodium stearyl fumarate, macrogols or mixtures thereof. A particularly preferred member of this group is magnesium stearate.

In a further embodiment the present invention provides for a pharmaceutical solid dosage formulation comprising the MPMS as defined above. The pharmaceutical solid dosage formulation may be in the form of a capsule, a tablet or a sachet. In a preferred embodiment, the solid dosage formulation is a capsule.

The number of particles which are contained in a solid dosage formulation depends on the strength of the dosage unit and the desired particle size. The MPMS in a preferred embodiment is a capsule, tablet or sachet containing more mini matrix solid uncoated particles. The number of said solid uncoated particles depends on the strength of the dosage unit. This means that the size of a capsule can, within certain limits, be selected independent from the strength and number of solid uncoated particles contained therein, as long as the capsule can host the particles needed to provide a certain dosage.

In a further embodiment, the present invention is directed to a process for the manufacture of a multi particulate matrix system as defined above. In this process each particle type of the MPMS is separately prepared by a process comprising a step selected from the group consisting of dry granulation, wet granulation, melt granulation and direct compression, followed by mixing of the respective separately obtained particle types.

"Dry granulation" means that powder components are mixed in an appropriate blender. The obtained homogenous mixture is agglomerated by roller compaction or slugging. The obtained compacts are crushed into granulate and if necessary sieved. Appropriate particle size fractions are used for manufacturing matrix tablets.

"Wet granulation" means that the powder obtained following the initial mixing of the components is granulated using proper quantities of granulation liquid. A drying step to remove the granulation liquid is necessary.

"Melt granulation" means a granulation process by which granules are obtained through the addition of either a molten binder or a solid binder which melts during the process. In the latter case the plastic properties of the binder are used. After the granulation the binder crystallizes at room temperature.

"Direct compression" involves the direct mixing of the dry components of the desired formulation, followed by a compression step to manufacture mini or micro tablets. The process does not involve the use of any liquid which may be the primary cause of instability of dosage forms and moreover requires an additional drying step to remove the granulation liquid in order to give the final dosage form. Therefore and also in view of cost aspects, direct compression is particularly preferred in this invention.

The step of direct compression, which is a preferred embodiment, includes the direct mixing of the dry components of the desired particle type, followed by a compression step to form a compact. A compact is to be understood as meaning particles in form of a micro or mini tablet having a diameter of 1 mm to 5 mm or less.

In a further embodiment the present invention relates to a process for forming a pharmaceutical solid dosage formulation as defined above comprising the step of melt granulation of a mixture containing at least the active ingredient and a binder to obtain granules, followed by either directly incorporation of these granules into capsules or sachets, or by compressing them into tablets.

### Examples

The following examples are to further illustrate preferred aspects of the invention.

Mini tablets (solid uncoated particles according to the present invention) were manufactured using a direct compression procedure (cf. Examples 1, 3, 4, 5). A wet granulation procedure was used in Comparative Example 2.

The average diameter of galantamine hydrobromide particles used in these Examples 1-5 was less than about 200 microns (D90 less than 250 microns/D50 less than about 100 microns).

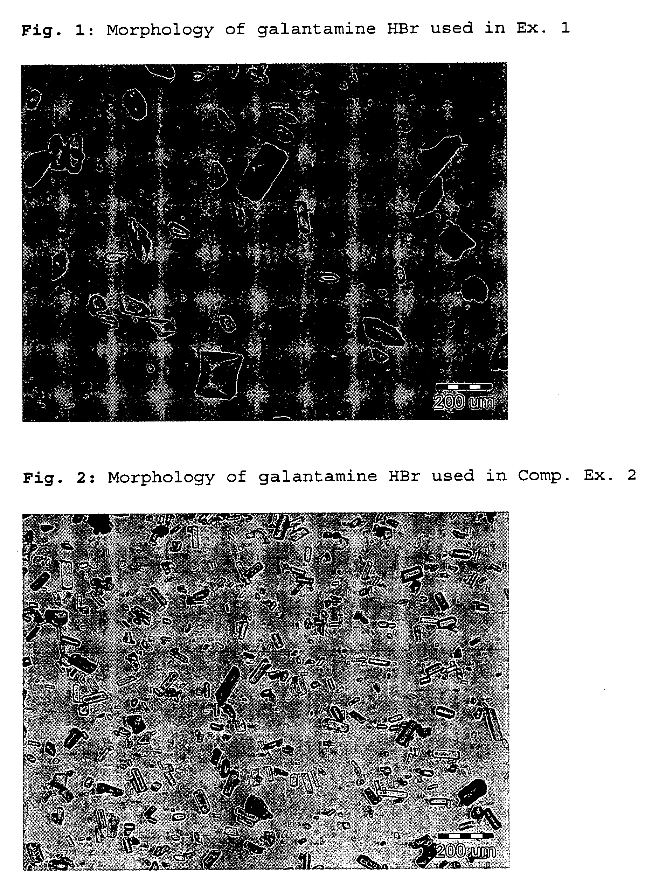

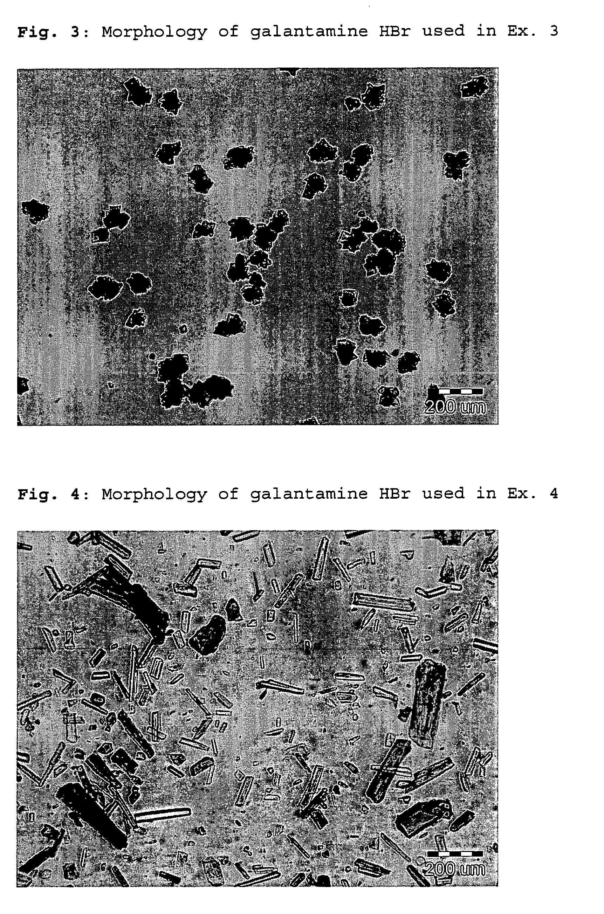

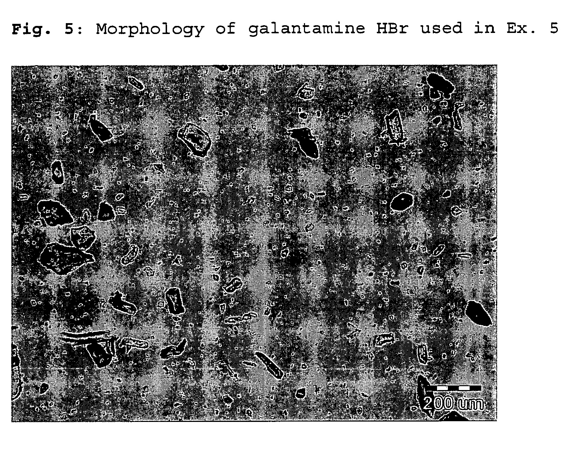

The morphology of the particles is shown in Figures 1-5. The microphotos were taken on Olympus microscope BX50 with magnitude of 100x.

The size distribution of galantamine hydrobromide particles was determined by laser diffraction. The method of determining the size of galantamine hydrobromide particles involved the use of a Malvern Mastersizer 2000 laser diffraction instrument. Samples for analysis were prepared by dispersing a weighed amount of galantamine hydrobromide in vegetable oil without sonication.

The following table shows the respective size distributions of the galantamine hydrobromide particles used in Examples 1-5:

**Table 1: Size distribution of galantamine HBr particles**

| | **Ex. 1 [µm]** | **Comp. Ex. 2 [µm]** | **Ex. 3 [µm]** | **Ex. 4 [µm]** | **Ex. 5 [µm]** |
|---|---|---|---|---|---|
| **D[3,4]** | 93 | 92 | 74 | 112 | 109 |
| **d10** | 62,8 | 6,2 | 15,4 | 29,4 | 16,6 |
| **d50** | 89,4 | 68,8 | 58,8 | 103,6 | 98,5 |
| **d90** | 127,2 | 215,6 | 153,2 | 205,3 | 215,4 |

### Examples 1&4

Galantamine HBr having a morphology and particle size distribution as shown above, Sodium Lauryl Sulphate, Eudragit RS PO, Methocel K4M , Kollidon SR, Magnesium stearate and talc in the amounts as indicated below were sieved through a 20 mesh sieve and mixed for an appropriate time period until a uniform mixture was formed. The resulting mixture was compressed using a rotary tabletting machine to give mini tablets (5 mm punches were used). For administration, these mini tablets were filled into gelatine capsules of size 2.

**Table 2: Composition of mini tablets obtained according to Examples 1&4**

| **Composition** | **Ex. 1 [mg]** | **Ex. 4 [mg]** |
|---|---|---|
| Galantamine Hydrobromide | 10.25 | 5.125 |
| Sodium Lauryl Sulphate | 3.00 | 4.72 |
| Eudragit RS PO | 6.30 | 7.44 |
| Methocel K4M | 21.90 | 23.025 |
| Kollidon SR | 6.30 | 7.44 |
| Magnesium stearate | 0.25 | 0.25 |
| Talc | 2.00 | 2.00 |
| Total | 50.00 | 50.00 |

### Examples 3&5

Galantamine HBr having a size distribution and morphology as described above, Tablettose, Methocel K4M and Magnesium stearate in the amounts as indicated below were sieved through a 20 mesh sieve and mixed for an appropriate time period until a uniform mixture was formed. The resulting mixture was compressed using a rotary tabletting machine to give mini tablets (5 mm punches were used). For administration, these mini tablets were filled into gelatine capsules of size 2.

**Table 3: Composition of mini tablets obtained according to Examples 3&5**

| **Composition** | **Ex. 3 [mg]** | **Ex. 5 [mg]** |
|---|---|---|
| Galantamine HBr | 10.25 | 5.125 |
| Tablettose | 14.00 | 16.875 |
| Methocel K4M | 25.25 | 27.50 |
| Magnesium stearate | 0.50 | 0.50 |
| Total | 50.00 | 50.00 |

### Comparative Example 2:

Galantamine HBr having a size distribution and morphology as described above, Sodium Lauryl Sulphate and Methocel K4M were sieved through a 20 mesh sieve and mixed in a mini high shear granulator for an appropriate time period until a uniform mixture was formed. Eudragit RS PO was dissolved in ethanol, giving a granulation liquid. The constituents of the internal phase were wet granulated using the granulation liquid. The granule mixture was dried to constant weight in a laboratory fluid bed. The dried granules were sieved and mixed with the rest of excipients and compressed. The resulting mixture was then compressed using a rotary tabletting machine to give mini tablets (5 mm punches were used). The mini tablets were filled into capsules size 2.

**Table 4: Composition of Comp. Ex. 2**

| **Composition** | **Comp. Ex. 2 [mg]** |
|---|---|
| Galantamine HBr | 10.25 |
| Sodium Lauryl Sulphate | 3.00 |
| Eudragit RS PO | 6.30 |
| Ethanol | * |
| Methocel K4M | 21.90 |
| Kollidon SR | 6.30 |
| Magnesium stearate | 0.25 |
| Talc | 2.00 |
| Total | 50.00 |

| | |
|---|---|
| *evaporates during the process | |

### Dissolution tests

After filling the mini tablets into gelatine size 2 capsules (in case of Examples 1 and 2 one mini tablet was filled in; in case of Examples 4 and 5 two mini tablets were filled in), the release profiles of the individual formulations of Examples 1, 4, and Comparative Example 2 respectively, were tested using 500 ml USP buffer pH 6.8 at 37°C in an Apparatus 1 (Ph.Eur. and USP, baskets, 100 rpm). The results of the dissolution tests are presented in the following table:

**Table 5: Release profile of mini tablets**

| **Dissolution Time / h** | **% dissolved** | | | |
|---|---|---|---|---|
| | **Ex.1** | **Comp. Ex.2** | **Ex.4** | **Ex.5** |
| 1 | 36 | 49 | 27 | 34 |
| 2 | 54 | 69 | 42 | 53 |
| 3 | 66 | 84 | 53 | 66 |
| 4 | 78 | 95 | 63 | 75 |
| 5 | 86 | 98 | 71 | 82 |
| 6 | 92 | 102 | 78 | 87 |
| 8 | 100 | 105 | 88 | 91 |
| 10 | 103 | 105 | 94 | 92 |
| 12 | 105 | 105 | 98 | 93 |
| 24 | 105 | 103 | 102 | 93 |

As already stated above, the dissolution values (%) obtained in the above dissolution profile have an accuracy of ±5% due to the nature of the preparation process of the mini and micro tablets.

From the above table it can be seen that the MPMS of Ex. 1 and Ex. 4 provide for a prolonged release whereas the MPMS of Comparative Ex. 2 provides for a faster release than for Ex. 1 and Ex. 4

Mini tablets prepared by Ex.1/Ex.4 comprise hydrophilic-lipophilic matrix polymers, while mini tablets prepared by Ex.3/Ex.5 only contain hydrophilic matrix polymers. Both types of polymers are satisfactory to form uncoated particle types that provided for prolonged release and can be used in a MPMS of the present invention. Moreover, the above Examples show that the size and shape of the active substance to be used to prepare a MPMS according to the present invention does not play any significant role in achieving stable formulation with prolonged release dissolution profile in comparison to other prolonged release systems.

## Claims

1. A multi particulate matrix system (MPMS) comprising at least one type of particles which contains galantamine or a pharmacologically acceptable salt thereof as an active substance,
wherein said at least one type of particles provides for prolonged release of said active substance,
and wherein all particles present in the MPMS are uncoated.

2. Multi particulate matrix system according to claim 1 which contains a second type of particles.

3. Multi particulate matrix system according to claim 2 wherein said second type of particles provides for either immediate release or prolonged release of the active substance.

4. Multi particulate matrix system according to any preceding claim, wherein the amount of active substance present in each particle type is 5-90% w/w.

5. Multi particulate matrix system according to any preceding claim, wherein the amount of active substance present in each particle type is 8-60% w/w.

6. Multi particulate matrix system according to any preceding claim, wherein the amount of active substance present in each particle type is 10-30% w/w.

7. Multi particulate matrix system according to any preceding claim wherein the particles are non-spherical.

8. Multi particulate matrix system according to claim 7, wherein the particles are in the form of micro or mini tablets having a diameter of 1 mm to 5 mm.

9. Multi particulate matrix system according to any preceding claim wherein the particles further comprise one or more pharmaceutically acceptable excipient(s) selected from the group consisting of a diluent, a binder, a disintegrant, a gelling agent, a non-swellable polymer, a lipid substance, a surfactant, a lubricant and a glidant.

10. Multi particulate matrix system according to claim 9, wherein
(a) the diluent is selected from the group consisting of microcrystalline cellulose, powdered cellulose, composite materials combining crystalline cellulose with lactose, guar gum or silicified cellulose, calcium hydrogen phosphate, lactose, fructose, dextranes, sugar alcohols, compressible or non-compressible sugars, calcium carbonate, calcium lactate or mixtures thereof,
(b) the binder is selected from the group consisting of polyvinylpyrrolidone, microcrystalline cellulose, cellulose ether, hydroxyethylcellulose, hydroxypropylcellulose, hydroxypropylmethylcellulose, starch, pregelatinised starch, polymethacrylate, or mixtures thereof,
(c) the disintegrant is selected from the group consisting of crospovidone, starch, pregelatinised starch, sodium starch glycollate, microcrystalline cellulose, carboxymethylcellulose sodium (CMC-Na) or calcium (CMC-Ca), cross-linked CMC-Na, polacrilin potassium, low-substituted hydroxypropylcellulose or mixtures thereof,
(d) the gelling agent is selected from the group consisting of hydroxypropylmethylcellulose, hydroxypropylcellulose, hydroxycellulose phthalate, poly(ethyleneoxide), polylactic acid, xanthan gum, alginates, sodium and calcium carboxymethylcellulose, carragheenan, carbomer, carbopol, methylhydroxyethylcellulose, propylhydroxyethylcellulose, polyhema, methylcellulose, alginates and other swellable polymers,
(e) the non-swellable polymer is selected from the group consisting of ethyl cellulose, cellulose acetate propionate, cellulose acetate, poly(ethyl acrylate, methyl methacrylate, trimethylammonioethyl methacrylate chloride)1:2:0.1, poly(ethyl acrylate, methyl methacrylate, trimethylammonioethyl methacrylate chloride) 1:2:0.2 copolymer, polyvinylpyrrolidone acetate, polyvinyl chloride, polyvinyl acetate, and polyethylene,
(f) the lipid substance is selected from the group consisting of fatty alcohols with 10-18 C atoms such as stearol, palmitol, esters and ether of fatty acids such as mono, di and triglycerides with 10-18 C atoms in the fatty acid residues,
(g) the surfactant is selected from the group consisting of anionic surfactants, ampholytic surfactants, nonionic surfactants and cationic surfactants,
(h) the lubricant and the glidants are selected from the group consisting of stearic acid, magnesium stearate, magnesium palmitate, magnesium oleate, hydrogenated vegetable oil, hydrogenated castor oil, talc, sodium stearyl fumarate, macrogols or mixtures thereof.

11. Multi particulate matrix system according to claim 1, wherein at least one particle type provides for a release profile of active ingredient such that after 2h 20 to 60% of the active substance originally contained is released, after 4h 40 to 80% of the active ingredient originally contained is released, and after 12h more than 70% of the active ingredient originally contained is released.

12. Multi particulate matrix system according to claim 1, wherein at least one particle type provides for a release profile of active ingredient such that after 12h, preferably after 16h, 80% or more of the active ingredient originally contained is released.

13. Multi particulate matrix system according to any preceding claim wherein the active substance is present in the form of a hydrate or solvate thereof.

14. Multi particulate matrix system according to any preceding claim wherein the active substance is galantamine hydrobromide.

15. A pharmaceutical solid dosage formulation comprising the multi particulate matrix system according to any of the preceding claims.

16. A pharmaceutical solid dosage formulation according to claim 15, which is in the form of a capsule, a tablet or a sachet.

17. A process for the manufacture of a multi particulate matrix system according to any of claims 1-14 wherein each particle type of the multi particulate matrix system is prepared by a process comprising a step selected from the group consisting of dry granulation, wet granulation, melt granulation and direct compression, followed by mixing of the respective particle types.

18. Process according to claim 17 wherein the step of direct compression includes the direct mixing of the dry components of the desired particle type, followed by a compression step to form a compact.

19. A process for forming a pharmaceutical solid dosage formulation according to claim 15 or 16 comprising the step of melt granulation of a mixture containing at least the active ingredient and a binder to obtain granules, followed by either directly incorporating these granules into capsules or sachets, or by compressing them into tablets.
